# EUROPEAN PATENT SPECIFICATION

(11) **EP 2 255 720 B1**
(45) Date of publication and mention of the grant of the patent: **18.12.2013**
(21) Application number: 10004638.2
(22) Date of filing: 03.05.2010
(51) Int. Cl.: A61B 1/01, A61B 1/273, B29C 45/00, B29C 55/00

(54) **Bending instrument for an endoscope and method of producing same**
Biegeinstrument für ein Endoskop und Verfahren zu dessen Herstellung
Instrument de pliage pour endoscope et son procédé de production

(30) Priority: 27.05.2009 JP 2009127211
(43) Date of publication of application: 01.12.2010
(73) Proprietor: Covidien LP, Mansfield, MA 02048 (US)
(72) Inventor: Abe, Kazuhiro, Shizuoka 437-0004 (JP)
(74) Representative: Olsson, Carl H.S.

(56) References cited:
- EP-A1- 2 074 931
- WO-A1-98/26724
- GB-A- 1 198 617
- US-A- 5 306 245

## Description

### [Technical Field]

The present invention relates to a bending instrument for an endoscope which is used for attachment to an endoscope in order to bend the tip end section of the endoscope, and to a method of producing the same.

### [Prior Art]

Internal organs such as the stomach wall are conventionally observed by inserting an endoscope nasally or orally into the stomach of a patient, but this method can cause discomfort to the patient. Consequently, it has become recent practice to make a gastrostomy catheter indwelling in a gastric fistula formed in the patient's body and to observe the inside of the stomach by passing an endoscope inside said gastrostomy catheter so as to confirm the indwelling position of the gastrostomy catheter. In such cases it is necessary to be able to change the orientation of the tip end of the endoscope for observation in various directions in order to accurately check the state inside the stomach and confirm the indwelling position of the gastrostomy catheter. Consequently, a bending instrument for an endoscope in which the direction of orientation of the tip end of the endoscope can be changed is used (see Patent Document 1, for example).

This bending sheath for a probe (bending instrument for an endoscope) has a configuration in which a helical cutaway part is provided towards the tip end of the sheath, and this section acts as an extension for detachably linking the main body portion of the sheath and the tip end holding part provided at the tip end thereof. A probe (endoscope) to which this bending sheath for a probe is fitted consists of an ultrasonic probe which is provided with an ultrasonic oscillator at the tip end, and said ultrasonic probe is connected to a device which can produce images of regions deep inside bodily cavities on an ultrasonic monitor by sending and receiving ultrasonic waves to and from the patient via the ultrasonic oscillator.

Consequently, the ultrasonic probe is inserted into the sheath and the tip end thereof runs into the inner wall of the tip end holding part, after which, with continued insertion of the ultrasonic probe, the portion at the tip end of the ultrasonic probe starts to bend so as to move away from the axial direction of the sheath. By means of this, the tip end of the ultrasonic probe can be oriented in the required direction, making it possible to display images of regions deep inside bodily cavities. [Patent Document 1] Japanese Unexamined Patent Application Publication 2001-120496.

Patent Document 2, WO 98/26724 shows a catheter assembly in which in order to provide a bending action a slot is made in a distal end of a sheath along an axial direction. The remaining portion of the sheath in the region of the slot is preferably reinforced, for example by thickening the material in this region.

### [Summary of the Invention]

However, with the bending sheath for a probe described above in respect of patent document 1, the extension is formed by providing the helical cutaway part on the sheath, and therefore there are problems in terms of the work involved in producing the extension and in producing the cutaway part accurately. Furthermore, the extension can extend elastically, and therefore there are problems in that it is difficult to set the length of the extension appropriately. Moreover, consideration has been given to joining the main body portion of the sheath and the tip end holding part using a thread, but this leads to strength problems in that stress is concentrated on the linking part, which makes it likely to break, and problems with the difficulty of manufacture.

The present invention has been devised in order to resolve the problems described above, and it aims to provide a bending instrument for an endoscope with which an extended linking part for joining a fixed part and a sliding part can be easily formed, while the length thereof can be freely set, and a method of producing the same.

In order to achieve the aim described above, a bending instrument for an endoscope has been defined in claim 6.

With the bending instrument for an endoscope according to the present invention, the fixed part, sliding part and linking part which joins the fixed part and the sliding part are first of all moulded as a single piece, after which the bending instrument for an endoscope is formed by extending the linking part to a specific length by drawing. The extended linking part in this case may be formed as various shapes, including a flexible cord-like shape, thread-like shape, rod-like shape, or a narrow sheet-like shape. For this reason, when the integral moulding is carried out, the linking part is formed into a shape which is simple to mould, such as a cylindrical column, a square column or an elongate sheet; after the moulding, an extended linking part of any diameter, width and shape etc. can be formed by drawing the linking part. The length of the extended linking part can also be freely set. In addition, the length of the extended linking part after drawing is substantially constant. This means that the gap between the fixed part and the sliding part can be restricted so that it does not exceed a specific length. Furthermore, in accordance with the present invention, the method of producing the bending instrument for an endoscope is simplified, and cost savings can be envisaged.

Further structural features of the bending instrument for an endoscope lie in the fact that the fixed part and the sliding part are formed to be annular or cylindrical, and specific portions on opposing end faces of the fixed part and the sliding part are joined by the extended linking part. In this case, it is possible to obtain a bending instrument for an endoscope which has a simple structure and good operability by making the fixed part and the sliding part annular or cylindrical.

Both end portions of the extended linking part in the longitudinal direction are thicker or wider than the central portion thereof. This means that the strength at the boundary sections of the extended linking part with the fixed part and the sliding part is increased, and therefore stress is not concentrated at the two ends of the extended linking part and so there is no breakage, thereby improving stability. Furthermore, the two end portions of the linking part in the longitudinal direction prior to the drawing are preferably also thicker or wider than the central portion. This means that stress is not concentrated at the two ends of the linking part and there is no breakage when the linking part undergoes drawing. It should be noted that the thickness and width of the linking part prior to drawing are substantially the same overall, and during the drawing the two end portions of the linking part in the longitudinal direction can be made thicker or wider than the central portion.

Further structural features of the bending instrument for an endoscope lie in the fact that it is used for the insertion of the endoscope into a gastrostomy catheter which is provided with a tubular part formed with an internal through-hole, and a stomach-internal fixed part which is linked to the tip end of the tubular part with the tip end of the through-hole of the tubular part remaining open and which is provided with an engaging part in the region of the tip end of the through-hole; the tubular part is positioned in a gastric fistula formed between the surface of a patient's skin and the inner surface of the stomach wall, and the gastrostomy catheter is made indwelling in the gastric fistula with the stomach-internal fixed part positioned inside the stomach; and the fixed part can pass through the inside of the gastrostomy catheter together with the endoscope, and the sliding part can pass through the through-hole of the gastrostomy catheter but engages with the engaging part of the stomach-internal fixed part in such a way that it cannot pass through the stomach-internal fixed part.

According to the present invention, the fixed part has a structure enabling it to pass through the inside of the gastrostomy catheter, and the sliding part has a structure enabling it to pass through the through-hole of the gastrostomy catheter, but it engages with the engaging part of the stomach-internal fixed part in such a way that it cannot pass through the stomach-internal fixed part. Accordingly, the endoscope to which the bending instrument for an endoscope has been attached is inserted into the gastrostomy catheter, and when the sliding part of the bending instrument for an endoscope reaches the engaging part of the stomach-internal fixed part, the sliding part engages with the engaging part. After this, when the endoscope is pushed further into the gastrostomy catheter, progression of the tip end of the endoscope in the direction of insertion is restricted by the fixed part, and it can only move together with the fixed part in the direction of an arc with the length of the extended linking part as the radius.

Furthermore, the portion of the endoscope further towards the base end than the tip end is pushed outwards at the tip end of the gastrostomy catheter, and therefore the tip end section of the endoscope projects outwards at the tip end of the gastrostomy catheter while bending. That is to say, this kind of operation is possible because corresponding parts of the fixed part and sliding part are joined by the extended linking part. In this case, the endoscope can rotate about an axis, and the insertion length of the endoscope can be adjusted, so that the tip end of the endoscope can be oriented in any direction. Furthermore, the fixed part is attached to the outer periphery of the tip end of the endoscope, and therefore there is no impediment to observation of the stomach wall etc. by the endoscope. This means that the direction of observation by the endoscope can be changed using a simple operation, and the state of the stomach wall and the indwelling position of the gastrostomy catheter can be more reliably confirmed.

Further structural features of the bending instrument for an endoscope lie in the fact that the endoscope is covered by a sheath, and the bending instrument for an endoscope is attached to the endoscope with the sheath interposed. In this case, the sheath may consist of a member which is able to cover at least the whole of the section of the endoscope which is inserted into the body. Furthermore, the tip end of the sheath comprises a light-transmissive window part or similar, and the observational accuracy of the stomach wall or the like by the endoscope is not reduced by the sheath. There is virtually no need to sterilize or clean the endoscope after it has been used because the sheath is employed, and costs involved in cleaning and sterilization are largely unnecessary, and it is possible to extend the lifespan of the endoscope.

Features of the method of producing a bending instrument for an endoscope lie in the fact that it is a method of producing a bending instrument for an endoscope which is used for attachment to an endoscope in order to bend the tip end section of the endoscope, said method including the features as defined in claim 1.

According to the present invention, it is possible to simply form a bending instrument for an endoscope provided with an extended linking part of suitable length. Furthermore, in this case, the endoscope is covered by a sheath when it is used, and the moulded body moulded in the moulding step is moulded from the same material as the material constituting the sheath, and a welding step may be carried out after the drawing step, in order to weld the fixed part of the bending instrument for an endoscope to the outer periphery of the tip end of the sheath. This means that the process for fixing the bending instrument for an endoscope to the sheath is simplified.

### [Brief Description of the Figures]

[Figure 1] is an oblique view showing the bending instrument for an endoscope according to a mode of embodiment of the present invention;
[Figure 2] shows the bending instrument for an endoscope, where (a) is a reverse view, and (b) is a view in cross section along 2-2 in (a);
[Figure 3] shows the moulded body prior to drawing, where (a) is a reverse view, and (b) is a view in cross section along 3-3 in (a);
[Figure 4] is a front view showing a state in which the bending instrument for an endoscope and an insertion aid are attached to the endoscope;
[Figure 5] is a front view showing the endoscope; [Figure 6] shows the gastrostomy catheter, where (a) is a plan view, (b) is a front view, and (c) is a bottom view;
[Figure 7] is an enlarged partial view in cross section showing a state in which the bending instrument for an endoscope has engaged with the cylindrical engaging part of the stomach-internal fixed part;
[Figure 8] is a partial cutaway in cross section showing a state in which the endoscope to which the bending instrument for an endoscope has been attached is positioned above the gastrostomy catheter which has been made indwelling in the body of a patient;
[Figure 9] is a partial cutaway in cross section showing a state in which the endoscope to which the bending instrument for an endoscope has been attached is inserted into the gastrostomy catheter which has been made indwelling in the body of the patient; and
[Figure 10] is a partial cutaway in cross section showing a state in which the indwelling position of the gastrostomy catheter is confirmed by the endoscope.

### [Mode of Embodiment of the Invention]

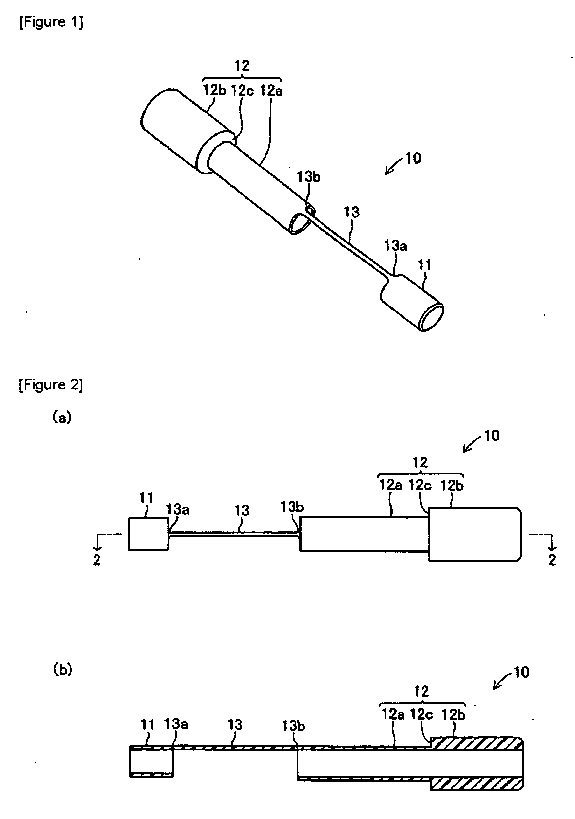

A mode of embodiment of the present invention will be described below with the aid of the figures. Figures 1 and 2 show a bending instrument 10 for an endoscope according to this mode of embodiment. This bending instrument 10 for an endoscope comprises a cylindrical fixed part 11, a stepped cylindrical sliding part 12 which is longer in the axial direction than the fixed part 11, and an extended linking part 13 for joining the fixed part 11 and sliding part 12. Hereinbelow, the side of the fixed part 11 of the bending instrument 10 for an endoscope shall be referred to as the lower side/bottom, and the side of the sliding part 12 shall be referred to as the upper side/top. The fixed part 11 which is positioned at the lower side of the bending instrument 10 for an endoscope comprises a cylindrical body.

The sliding part 12 comprises a sliding part main body 12a which is positioned at the bottom, and a latch part 12b which is positioned at the top. The sliding part main body 12a comprises a cylindrical body the diameter of which is set to be substantially the same as the diameter of the upper end of the fixed part 11, and the length of which is greater than that of the fixed part 11. Furthermore, the latch part 12b comprises a cylindrical body having a larger diameter than the sliding part main body 12a. A step 12c is then formed at the lower end of the latch part 12b. Furthermore, the extended linking part 13 joins opposing portions at the top end edge of the fixed part 11 and the bottom end edge of the sliding part main body 12a, and it is flexible. Expanded-width parts 13a, 13b which are wider than the central portion of the extended linking part 13 are then formed at the two ends of the extended linking part 13.

The expanded-width part 13a is formed by providing concave curves which grow steadily greater in width from the centre of the extended linking part 13 towards the fixed part 11 on both sides of the extended linking part 13 in the width direction (the peripheral direction of the fixed part 11) at the boundary between the fixed part 11 and the extended linking part 13. Likewise, the expanded-width part 13b is formed by providing concave curves which grow steadily greater in width from the centre of the extended linking part 13 towards the sliding part main body 12a on both sides of the extended linking part 13 in the width direction (the peripheral direction of the sliding part main body 12a) at the boundary between the sliding part main body 12a and the extended linking part 13.

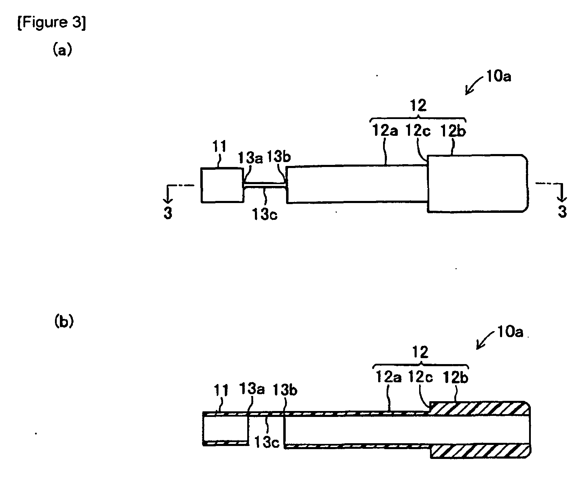

This bending instrument 10 for an endoscope is produced in the following manner. First of all, an injection moulding apparatus (not depicted) provided with a die formed with a moulding cavity in the shape of the moulded body 10a shown in Figure 3 is used to produce the moulded body 10a by injection moulding a moulding material comprising polypropylene or polyethylene or similar under conditions of temperature 200 - 250°C and injection moulding pressure 25 - 40 MPa. This moulded body 10a has a linking part 13c for joining the fixed part 11 and sliding part 12 in a pre-drawn state, and the linking part 13c is thicker and shorter than the extended linking part 13 of the bending instrument 10 for an endoscope.

The lengths of the sections of the moulded body 10a in the axial direction are set at 3 mm for the fixed part 11, 4 mm for the linking part 13c, 13 mm for the sliding part main body 12a, and 10 mm for the latch part 12b, and the overall length is 30 mm, for example. Furthermore, r (radii) of the concave curves of the expanded-width parts 13a, 13b are set at 0.5 mm. In addition, the fixed part 11 has an outer diameter of 2.53 mm and an inner diameter of 2.03 mm, and the width of the linking part 13c is 0.4 mm. Furthermore, the sliding part 12 has an inner diameter of 2.08 mm, and the outer diameter of the sliding part main body 12a is set at around 2.77 mm, while the outer diameter of the latch part 12b is set at around 3.8 mm. These dimensions are suitably set to fit the dimensions of the endoscope 15 and sheath 14 which will be described later.

Next, the linking part 13c of this moulded body 10a is drawn using a specific drawing apparatus (not depicted). This drawing apparatus is provided with two fixed parts which can advance and retract with respect to each other. For these two fixed parts, it is possible to use a system in which a fixed static plate-like fixed part and a mobile plate-like fixed part which can advance and retract with respect to the static plate-like fixed part are arranged facing each other, and a recess allowing the linking part 13c to pass through is formed in the respective upper edges thereof. In this case, the opposing end faces of the fixed part 11 and the sliding part 12 are brought into contact with the outside faces of the static plate-like fixed part and the mobile plate-like fixed part, respectively. The fixed part 11 and the sliding part 12 are then fixed to the two fixed parts, and the two fixed parts are steadily moved apart to draw the linking part 13c to a specific length, 13 mm, for example.

The rate at which the two fixed parts are moved apart in this instance is set to around 1 mm/sec. Furthermore, the deformation of the linking part 13c when it is drawn is achieved through plastic deformation and elastic deformation. That is to say, the linking part 13c slightly contracts from immediately after the end of drawing to become the extended linking part 13, after which the length thereof is maintained. For this reason, the drawing is carried out for a slightly longer time than the 9 seconds required in order to draw a linking part 13c of length 4 mm to 13 mm, for example approximately 10 - 13 seconds. Next, the bending instrument 10 for an endoscope obtained by drawing is sterilized. This sterilization is carried out by placing the bending instrument 10 for an endoscope in ethylene oxide gas at 40 - 50°C for around 20 hours.

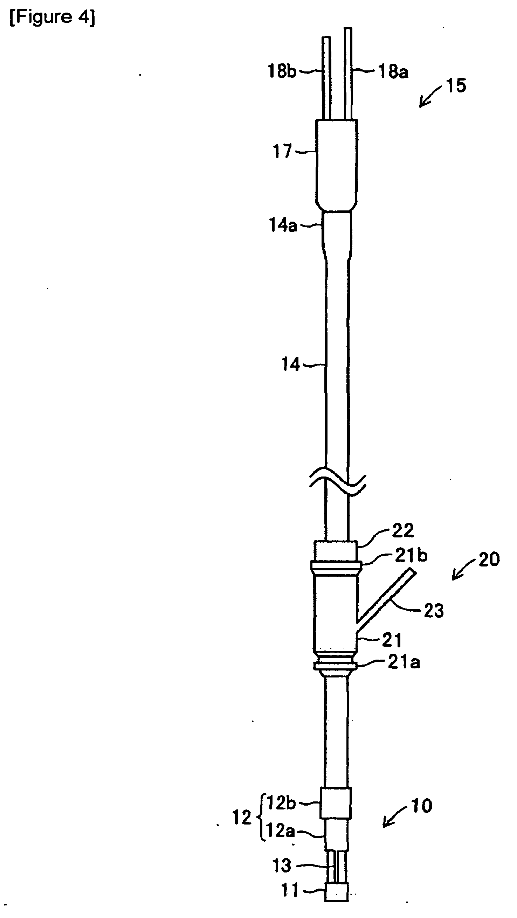

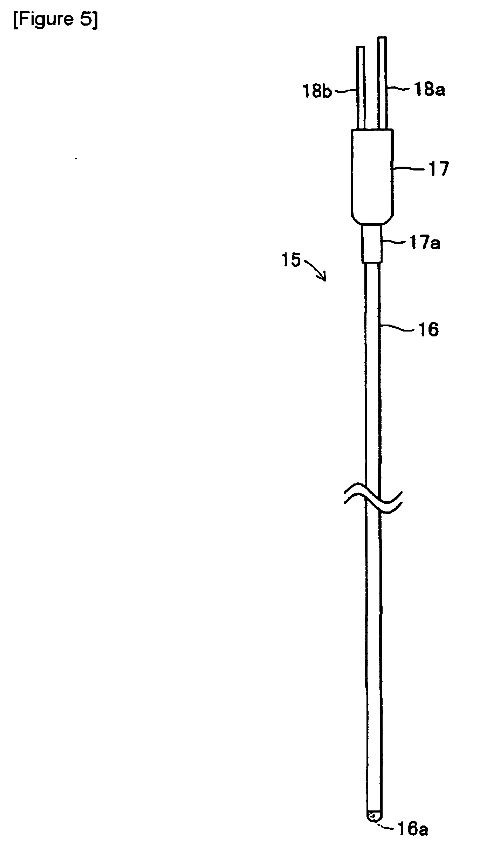

The bending instrument 10 for an endoscope obtained in this manner is attached to the endoscope 15 with the sheath 14 interposed, as shown in Figure 4. That is to say, the sheath 14 covers the fibrescope shaft 16 (see Figure 5) of the endoscope 15, which will be described later, in order to prevent soiling of the fibrescope shaft 16; the bending instrument 10 for an endoscope is used to make the tip end section of the fibrescope shaft 16 bend so that the direction of observation by the endoscope 15 can be changed. The endoscope 15 has a configuration in which a lens 16a is attached to the tip end of the fibrescope shaft 16, and a connector 17 is attached to the rear end, as shown in Figure 5.

The fibrescope shaft 16 is flexible and it consists of a bundle of fibres comprising a light guide for illuminating light, and an image guide for transmitting reflected light via the lens 16a. The connector 17 is joined to wiring 18a which connects the image guide to an image display device (not depicted), and wiring 18b which connects the light guide to a light source device (not depicted). The tip end of the sheath 14 is closed off by the light-transmissive window part (not depicted), and the base end 14a thereof at the open side comprises a tube of somewhat larger diameter than the other sections; the sheath is also flexible.

The sheath 14 is attached to the fibrescope shaft 16 by pushing the tip-end narrow-diameter part 17a of the connector 17 into the base end 14a. In this case, the sheath 14 is prevented from being removed from the fibrescope shaft 16 using a member such as a clamp, binding or clasp. In this state, the lens 16a is designed to come into contact with the inner surface of the window part. The sheath 14 is made of the same material as the material which constitutes the bending instrument 10 for an endoscope, and the sheath is inserted from the top opening of the bending instrument 10 for an endoscope; the sheath is made integral with the bending instrument 10 for an endoscope by welding the tip end at the outer periphery of the sheath to the inner peripheral surface of the fixed part 11. The welding in this case is preferably heat welding.

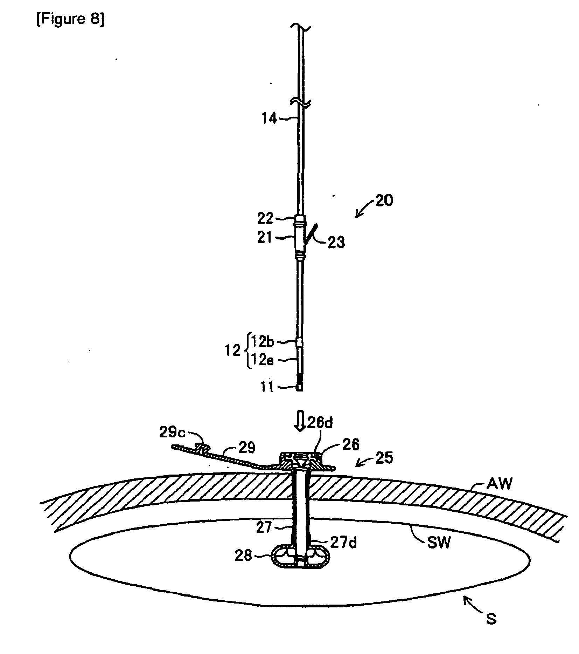

Furthermore, an insertion aid 20 is attached to the top part of the bending instrument 10 for an endoscope at the outer periphery of the sheath 14. The insertion aid 20 is an instrument which is fitted to the gastrostomy catheter 25 (to be described later) in order to smooth the operation to insert the endoscope 15 etc. into the gastrostomy catheter 25, and also to supply air into the stomach S (see Figures 8 to 10) so as to expand the stomach S, and it comprises a cylindrical main body 21, a valve restraining member 22, a disc-shaped sealing member with a hole (not depicted), and a branch pipe 23 which branches off from the cylindrical main body 21. The cylindrical main body 21 is formed as a cylinder in which is formed a through-hole which allows the passage of the fibrescope shaft 16 together with the sheath 14. Furthermore, a connector 21a is formed at the lower end of the cylindrical main body 21, and an insertion opening 21b is formed at the upper end of the cylindrical main body 21.

The connector 21a has a configuration in which a large-diameter section of substantially the same diameter as the central section of the cylindrical main body 21 is formed in substantially the centre in the axial direction of a narrow-diameter section which is narrower in diameter than the central section of the cylindrical main body 21. The insertion opening 21b is formed with a larger diameter than the central section of the cylindrical main body 21. Furthermore, the top part of the insertion opening 21b is narrower in diameter than the bottom part thereof, and an engaging part with which the valve restraining member 22 engages is formed on the outer peripheral surface thereof, although this is not shown in the figures. The valve restraining member 22 comprises a cap-like body formed with a ceiling part and a hole part, and a sealing member is housed therein.

The sealing member comprises a deformable annular elastomer, for example natural rubber, synthetic rubber, silicone or the like, and the inner diameter thereof is somewhat smaller than the inner diameter of the through-hole of the cylindrical main body 21, and the outer diameter thereof is substantially the same as the outer diameter of the upper end face of the insertion opening 21b. Furthermore, the branch pipe 23 is formed as a cylinder which extends obliquely upwards from the upper side of the connector 21a on the cylindrical main body 21, at an inclination of approximately 45° with respect to the cylindrical main body 21, and it is narrower in diameter than the cylindrical main body 21. An air supply device (not depicted) is connected to the tip end of this branch pipe 23, and air which is supplied by the air supply device passes through the branch pipe 23 and is fed into the cylindrical main body 21.

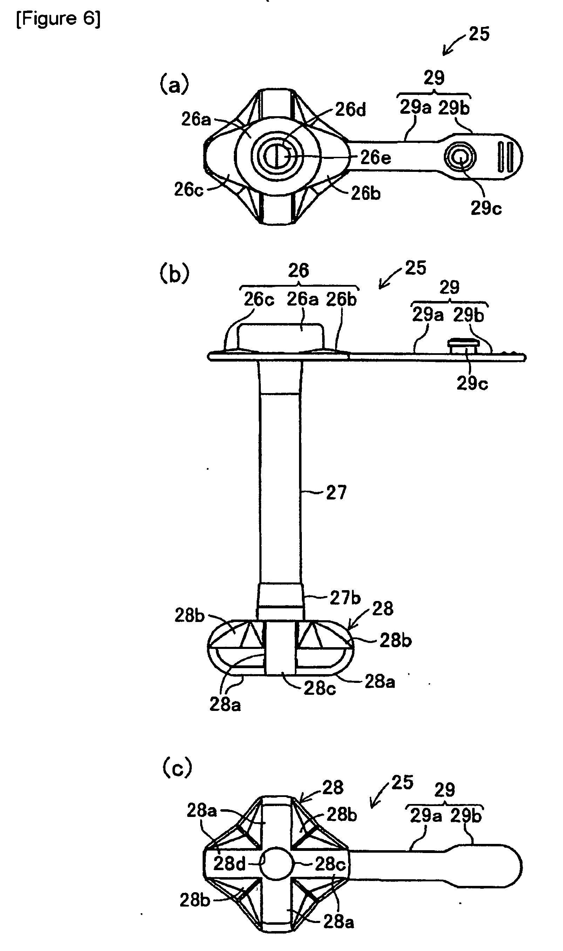

As shown in Figure 6, the gastrostomy catheter 25 comprises an external fixed part 26, a tubular part 27 which is linked to the centre of the lower end surface of the external fixed part 26, and a stomach-internal fixed part 28 which is attached to the lower end of the tubular part 27, all these components being made of a soft plastic material such as polyurethane or silicone. The external fixed part 26 comprises an insertion opening 26a which is formed as a thick ring, and projecting pieces 26b, 26c which project at both sides from the lower end of both side parts of the insertion opening 26a. A valve body 26e which is formed with a central slit is then provided on the inner peripheral surface of an insertion hole 26d which is formed in the centre of the insertion opening 26a, passing through vertically.

Furthermore, a cover part 29 for closing off the insertion hole 26d of the insertion opening 26a is joined to the tip end of the projecting piece 26b. The cover part 29 comprises an elongate strip-shaped linking part 29a which is linked to the projecting piece 26b, a broad part 29b which is short and wide, and is formed at the tip end of the strip-shaped linking part 29a, and a stopper part 29c shaped like a round column which is short in the axial direction and is provided on the broad part 29b. The stopper part 29c is provided on the broad part 29b, so as to face the insertion hole 26d when the strip-shaped linking part 29a is bent to position the broad part 29b above the insertion opening 26a.

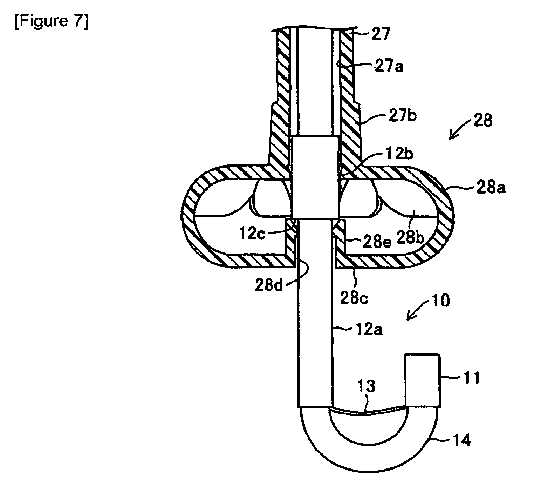

The tubular part 27 comprises an elongate cylindrical body inside which is formed a through-hole 27a (see Figure 7) for allowing the passage of fluids such as nutrients and food in fluid form, and the upper end of the through-hole 27a communicates with the insertion hole 26d of the external fixed part 26. The stomach-internal fixed part 28 is connected to the tubular part 27 by way of a connector 27b which is fixed to the lower end of the tubular part 27. This stomach-internal fixed part 28 comprises four strip-shaped linking parts 28a which extend in four directions from the edge of a lower end opening of the connector 27b, four linking film parts 28b which are provided between the upper parts of each of the linking parts 28a and form a stomach wall contact part with the four linking parts 28a, and a converging part 28c where the tip ends of all of the linking parts 28a converge.

The four linking parts 28a comprise strip-shaped members which are bent into substantially semi-circular shapes which split into four directions from the lower end of the connector 27b, respectively extending downwards from the horizontal, after which they converge below the central axis of the tubular part 27, linking to form the converging part 28c. Furthermore, each of the linking parts 28a and each of the linking film parts 28b is made of a flexible soft elastic material, and the overall shape thereof is normally maintained as a flat and substantially spherical shape, as shown in Figure 6(b), but they can be extended into a straight elongate shape by pulling the converging part 28c downwards. Furthermore, the lower end of the insertion hole 27a in the tubular part 27 opens between the upper ends of the linking parts 28a.

The space formed between the bottom parts of the linking parts 28a forms a channel for the passage of fluids such as nutrients and food in fluid form delivered from the through-hole 27a in the tubular part 27, into the stomach S. A further through-hole 28d is formed in the centre of the converging part 28c, and a cylindrical engaging part 28e (see Figure 7) which can engage with the step 12c of the bending instrument 10 for an endoscope is formed at the top part of the through-hole 28d (converging part 28c). That is to say, the through-hole 28d is constituted by the inner peripheral surface of the cylindrical engaging part 28e, and the diameter thereof is smaller than the diameter of the insertion hole 26d in the external fixed part 26 and the diameter of the through-hole 27a in the tubular part 27.

Moreover, the outer diameters of the fixed part 11 and the sliding part main body 12a of the bending instrument 10 for an endoscope are smaller than the diameter of the through-hole 28d of the stomach-internal fixed part 28. Furthermore, the outer diameter of the step 12c is smaller than the diameters of the through-hole 26d of the external fixed part 26 and the through-hole 27a of the tubular part 27, but greater than the diameter of the through-hole 28d of the stomach-internal fixed part 28. Consequently, when the endoscope 15 to which the sheath 14 and bending instrument 10 for an endoscope have been attached is inserted into the gastrostomy catheter 25 from the insertion hole 26d towards the through-hole 28d, the bending instrument 10 for an endoscope passes through the insertion hole 26d of the external fixed part 26 and the through-hole 27a of the tubular part 27.

The fixed part 11 and the sliding part main body 12a can then pass through the inside of the gastrostomy catheter 25 from the insertion hole 26d towards the through-hole 28d, but when the step 12c of the sliding part 12 reaches the cylindrical engaging part 28e, the step 12c comes into contact with the cylindrical engaging part 28e, and the latch part 12b cannot pass through the through-hole 28d. If the endoscope 15 and other members are inserted further in this state, the tip end section of the endoscope 15 bends, as shown in Figure 7, and the orientation of the tip end of the endoscope 15 is changed. In this case, the extended linking part 13 is positioned at the inner periphery of the fibrescope shaft 16 and other members when they are bent, and the distance between the upper end edge of the fixed part 11 (the lower end in Figure 7) and the lower end edge of the sliding part 12 remains substantially constant.

Furthermore, the connector 21a of the insertion aid 20 is designed to be able to be connected to the insertion hole 26d of the gastrostomy catheter 25 in an airtight and liquid-tight manner. Furthermore, the connector 21a pushes the slit of the valve body 26e formed in the insertion hole 26d outwards at this point so that the outer peripheral surface of the connector 21a and the peripheral edge of the slit are in very close contact. In addition, when the insertion aid 20 is attached to the fibrescope shaft 16 which is covered by the sheath 14, the area between the sealing member and the sheath 14 is in airtight and liquid tight contact.

A description will be given next of the method of confirming the indwelling position of the gastrostomy catheter 25 and the state of the inside of the stomach S by using the endoscope 15 to which the bending instrument 10 for an endoscope, sheath 14 and insertion aid 20 have been attached, with the aid of Figures 8 to 10. Figure 8 shows a state in which the gastrostomy catheter 25 is indwelling in a gastric fistula provided in the patients' abdominal wall AW and stomach wall SW, and in the state shown in Figure 8, the stopper part 29c of the gastrostomy catheter 25 is removed from the insertion hole 26d so that the top end of the insertion hole 26d is open. Furthermore, the endoscope 15 to which the bending instrument 10 for an endoscope, sheath 14 and insertion aid 20 have been attached is positioned above the gastrostomy catheter 25.

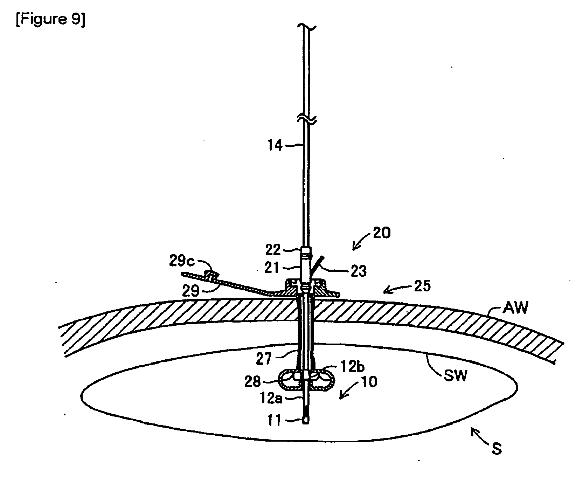

The endoscope 15 and other members which are in this state are moved downwards as shown by the arrow in the figure so that the fibrescope shaft 16 protruding from the lower end of the insertion aid 20 is inserted into the insertion hole 26d of the gastrostomy catheter 25 together with the sheath 14 and bending instrument 10 for an endoscope. By means of this, the connector 21a of the insertion aid 20 engages with the insertion hole 26d of the gastrostomy catheter 25, as shown in Figure 9. The endoscope 15 and other members are then inserted further towards the lower side of the gastrostomy catheter 25, and the lower section of the fibrescope shaft 16, sheath 14 and bending instrument 10 for an endoscope are made to project downwards from the through-hole 28d of the gastrostomy catheter 25.

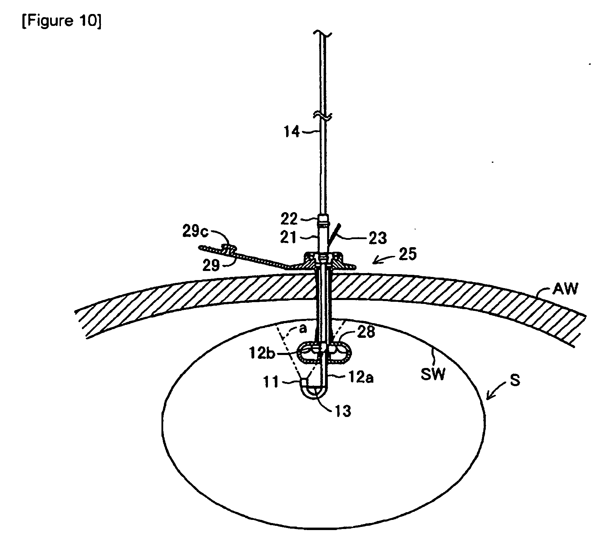

Next, air is supplied from the air supply device to the branch pipe 23, and this air is fed into the stomach S from the connector 21a through the tubular part 27 of the gastrostomy catheter 25. By means of this, the stomach S expands, as shown in Figure 10. In this state, light is produced by the light source device, whereby the light passes through the wiring 18b and the light guide of the fibrescope shaft 16, shining light towards the stomach wall SW. Furthermore, as shown in Figure 10, the lower section of the fibrescope shaft 16 may, in this case, be bent to change the position of illumination of the stomach wall SW by the light guide, by pushing the endoscope 15 and other members further into the body, if this is required.

That is to say, if the fibrescope shaft 16 is pushed further in with respect to the bending instrument 10 for an endoscope from the state shown in Figure 9, the fixed part 11 of the bending instrument 10 for an endoscope and the sliding part main body 12a of the sliding part 12 pass through the through-hole 28d of the stomach-internal fixed part 28, as shown in Figure 7, and enter the stomach S, but the engaging part 12b of the sliding part 12 comes into contact with the cylindrical engaging part 28e and is held inside the stomach-internal fixed part 28. Consequently, the fixed part 11 moves so as to describe an arc with the extended linking part 13 as the radius thereof, and the tip end sections of the fibrescope shaft 16 and sheath 14 protrude inside the stomach S while bending to follow the movement of the fixed part 11.

The range shown by the two-dot chain line a in Figure 10 shows the range of illumination of the light from the light guide. The reflected light from the stomach wall SW which is illuminated by the light guide is then focused by the lens 16a, after which it is sent to the image display device via the image guide of the fibrescope shaft 16 and the wiring 18a. The images which have been sent to the image display device are enlarged and displayed on the display part of the image display device, which makes it possible to confirm from these images displayed on the image display part whether or not the stomach-internal fixed part 28 of the gastrostomy catheter 25 is correctly arranged inside the stomach S, and to confirm the state of the stomach S. If it can be confirmed that the gastrostomy catheter 25 is indwelling in the correct state and the state of the stomach S can be confirmed, the endoscope 15 and other members are withdrawn.

This withdrawal operation involves pulling the endoscope 15 and other members slightly upwards, and once the state in Figure 9 has been reached, the engagement between the insertion aid 20 and gastrostomy catheter 25 is released. The endoscope 15 and other members are then pulled upwards to remove them from the gastrostomy catheter 25. The insertion aid 20 is then removed from the sheath 14, after which the fibrescope shaft 16 is withdrawn from the sheath 14. The bending instrument 10 for an endoscope and the sheath 14 are then discarded, and the endoscope 15 can be used on another occasion. Furthermore, when the endoscope 15 is reused, a sheath 14 to which a new bending instrument 10 for an endoscope is attached is employed.

Furthermore, when liquid nutrients, for example, are supplied to the patient's stomach S through the gastrostomy catheter 25 which has been made indwelling in the patient's body, a connector of a tube extending from a container housing the nutrients is connected to the insertion hole 26d of the gastrostomy catheter 25, and the nutrients are supplied to the patient through the tube and the gastrostomy catheter 25. Furthermore, the tube of the container housing the nutrients is removed from the insertion hole 26d of the gastrostomy catheter 25 after use, and the insertion hole 26d is closed with the stopper part 29c.

In this way, the bending instrument 10 for an endoscope according to this mode of embodiment is obtained by first of all injection moulding the moulded body 10a which comprises the fixed part 11, sliding part 12 and linking part 13c for joining the fixed part 11 and sliding part 12, after which the linking part 13c of the moulded body 10a is drawn to a specific length by drawing to form the extended linking part 13. In this way, the moulded body 10a having the short and thick linking part 13c is moulded as a provisional moulding, rather than the bending instrument 10 for an endoscope with the elongate extended linking part 13 which is difficult to injection mould being injection moulded from the outset, and therefore the moulded body 10a can be easily moulded. Furthermore, the linking part 13c can be easily drawn, and the length of the resulting extended linking part 13 can be freely set. Cost savings can also be envisaged.

Furthermore, the two end sections of the linking part 13c of the moulded body 10a in the longitudinal direction comprise the expanded-width parts 13a, 13b which are wider than the other sections, and therefore the strength at the boundary sections of the linking part 13c with the fixed part 11 and the sliding part 12 is increased, and the linking part 13 can be easily drawn. In addition, when the assembly is used, the endoscope 15 to which the bending instrument 10 for an endoscope has been attached is inserted into the gastrostomy catheter 25, and the engaging part 12b of the sliding part 12 engages with the cylindrical engaging part 28e; if the endoscope 15 is then pushed further into the gastrostomy catheter 25, the progression of the tip end of the endoscope 15 in the direction of insertion is restricted by the fixed part 11, and it can only move together with the fixed part 11 in the direction of an arc with the length of the extended linking part 13 as the radius.

The section of the endoscope 15 further towards the base end than the tip end is then pushed outwards at the tip end of the gastrostomy catheter 25, and therefore the endoscope 15 protrudes outwards at the tip end of the gastrostomy catheter 25 while bending. Consequently, the endoscope 15 can rotate about an axis, and the insertion length of the endoscope 15 can be adjusted, so that the tip end of the endoscope 15 can be oriented in any direction. Furthermore, the fixed part 11 is attached to the outer periphery of the tip end of the endoscope 15, and therefore there is no impediment to observation of the stomach wall SW etc. by the endoscope.

This means that the direction of observation by the endoscope 15 can be changed using a simple operation, and the state of the stomach wall SW and the indwelling position of the gastrostomy catheter 25 can be more reliably confirmed. Furthermore, the bending instrument 10 for an endoscope according to this mode of embodiment is fixed to the tip end of the sheath 14, and then attached to the endoscope 15 together with the sheath 14, and therefore there is virtually no need to sterilize or clean the endoscope 15 after it has been used, and costs involved in cleaning and sterilization are largely unnecessary, and it is possible to extend the lifespan of the endoscope 15.

Furthermore, the bending instrument for an endoscope according to this mode of embodiment is not limited to the mode of embodiment described above, and suitable modifications may be made within the technical scope of the present invention. For example, in the mode of embodiment described above, the bending instrument 10 for an endoscope is fixed to the sheath 14 by welding, but it may be fixed by bonding or the like rather than by welding, or the bending instrument 10 for an endoscope may be detachable from the sheath 14. Furthermore, the sheath 14 may be omitted, and the bending instrument 10 for an endoscope may be attached directly to the endoscope 15.

In addition, the extended linking part 13 may have various shapes such as a cord-like shape, thread-like shape, rod-like shape or a narrow sheet-like shape. Furthermore, the length of the extended linking part 13 may be freely set, and if the length of the linking part 13c is set at 4 mm, as in the mode of embodiment described above, the length of the extended linking part 13 may be 4 - 13 mm. In addition, the shapes of the other components which make up the bending instrument 10 for an endoscope may be suitably modified. Furthermore, the drawing of the linking part 13c may involve hot drawing at a specific temperature.

### [Key to Symbols]

10...bending instrument for an endoscope; 10a...moulded body; 11...fixed part; 12...sliding part; 12b...latch part; 12c...step; 13...extended linking part; 13a, 13b...expanded-width parts; 13c...linking part; 14...sheath; 15...endoscope; 25...gastrostomy catheter; 27...tubular part; 27a...through-hole; 28...stomach-internal fixed part; 28d...through-hole; 28e...cylindrical engaging part; AW...abdominal wall; S...stomach; SW...stomach wall.

## Claims

1. Method of producing a bending instrument (10) for an endoscope which is used for attachment to an endoscope in order to bend the tip end section of the endoscope,
said method being **characterized in that** it includes:
a moulding step in which a fixed part (11) which is attached to the outer periphery of the tip end of the endoscope, a sliding part (12) which is slidably attached further towards the base end of the endoscope than the portion where the fixed part is attached, and a linking part (13) for joining specific opposing portions of the fixed part and the sliding part are moulded as a single piece by injection moulding; and a drawing step in which the linking part of the moulded body moulded in the moulding step is formed into a flexible extended linking part for restricting the gap between the fixed part and the sliding part so that it does not exceed the specific length, by extending the linking part to a specific length by drawing such that both end portions of the extended linking part in the longitudinal direction are thicker or wider than the central portion thereof.

2. The method according to Claim 1, in which the endoscope is covered by a sheath (14) when it is used, and the moulded body moulded in the moulding step is moulded from the same material as the material constituting the sheath, and a welding step is carried out after the drawing step, in order to weld the fixed part of the bending instrument for an endoscope to the outer periphery of the tip end of the sheath.

3. The method according to claim 1, wherein the fixed part and the sliding part are formed to be annular or cylindrical, and specific portions on opposing end faces of the fixed part and the sliding part are joined by the extended linking part.

4. The method according to any preceding claim wherein the endoscope is suitable for insertion into a gastrostomy catheter which is provided with a tubular part formed with an internal through-hole, and a stomach-internal fixed part which is linked to the tip end of the tubular part with the tip end of the through-hole of the tubular part remaining open and which is provided with an engaging part in the region of the tip end of the through-hole; the tubular part is positioned in a gastric fistula formed between the surface of a patient's skin and the inner surface of the stomach wall, and the gastrostomy catheter is made indwelling in the gastric fistula with the stomach-internal fixed part positioned inside the stomach; and the fixed part can pass through the inside of the gastrostomy catheter together with the endoscope, and the sliding part can pass through the through-hole of the gastrostomy catheter but engages with the engaging part of the stomach-internal fixed part in such a way that it cannot pass through the stomach-internal fixed part.

5. The method according to any preceding claim, wherein the endoscope is covered by a sheath, and the bending instrument for an endoscope is attached to the endoscope with the sheath interposed.

6. Bending instrument (10) for an endoscope which is used for attachment to the endoscope in order to bend the tip end section of the endoscope,
said bending instrument for an endoscope comprising a fixed part (11) which is attachable to the outer periphery of the tip end of the endoscope, a sliding part (12) which in use is slidably attached further towards the base end of the endoscope than the portion where the fixed part is attached, and a flexible linking part (13) for joining specific opposing portions of the fixed part and the sliding part, the fixed part, the sliding part and the linking part be formed as a single piece and the gap between the fixed part and the sliding part can be restricted by the extended linking part so that it does not exceed the specific length, **characterized in that** the flexible linking part is formed by extending the linking part to a specific length by drawing and it is thinner at a central portion thereof than each end portion, the linking part, the fixed part and the sliding part are moulded as a single piece.

7. Bending instrument for an endoscope according to Claim 6, in which the fixed part and the sliding part are annular or cylindrical, and specific portions on opposing end faces of the fixed part and the sliding part are joined by the flexible linking part.

8. Bending instrument for an endoscope according to any one of Claims 6 to 7, in combination with an endoscope (15) and a gastrostomy catheter (25) for the insertion of the endoscope into the gastrostomy catheter wherein the gastrostomy catheter is provided with a tubular part (27) formed with an internal through-hole, and a stomach-internal fixed part (28) which is linked to the tip end of the tubular part with the tip end of the through-hole of the tubular part remaining open and which is provided with an engaging part in the region of the tip end of the through-hole; the tubular part is positioned in a gastric fistula formed between the surface of a patient's skin and the inner surface of the stomach wall, and the gastrostomy catheter is suitable for indwelling in the gastric fistula with the stomach-internal fixed part positioned inside the stomach; and
the fixed part can pass through the inside of the gastrostomy catheter together with the endoscope, and the sliding part can pass through the through-hole of the gastrostomy catheter but engages with the engaging part of the stomach-internal fixed part in such a way that it cannot pass through the stomach-internal fixed part.

9. Bending instrument for an endoscope according to any one of Claims 6 to 7 in combination with an endoscope (15), in which the endoscope is covered by a sheath (14), and the bending instrument for an endoscope is attached to the endoscope with the sheath interposed.

## Patentansprüche

1. Verfahren zur Herstellung eines Biegeinstruments (10) für ein Endoskop, das zum Anbringen an einem Endoskop verwendet wird, um den Spitzenendabschnitt des Endoskops zu biegen,
**dadurch gekennzeichnet, dass** es Folgendes aufweist:
einen Formschritt, bei dem ein feststehender Teil (11), der am äußeren Umfang des Spitzenendes des Endoskops angebracht ist, ein Schiebeteil (12), der weiter zum Basisende des Endoskops hin als der Abschnitt, an dem der feststehende Teil angebracht ist, schiebbar angebracht ist, und ein Verbindungsteil (13) zum Verbinden bestimmter gegenüberliegender Abschnitte des feststehenden Teils und des Schiebeteils einteilig durch Spritzgießen geformt werden, und
einen Ziehschritt, bei dem der Verbindungsteil des im Formschritt geformten Formkörpers zu einem flexiblen verlängerten Verbindungsteil zur Beschränkung des Spalts zwischen dem feststehenden Teil und dem Schiebeteil, so dass er die spezifische Länge nicht überschreitet, geformt wird, indem der Verbindungsteil durch Ziehen auf eine spezifische Länge verlängert wird, so dass beide Endabschnitte des verlängerten Verbindungsteils in der Längsrichtung dicker oder breiter als sein Mittelabschnitt sind.

2. Verfahren nach Anspruch 1, wobei das Endoskop im Gebrauch mit einer Hülle (14) bedeckt ist und der im Formschritt geformte Formkörper aus demselben Material wie das Material, aus dem die Hülle besteht, geformt ist, und nach dem Ziehschritt ein Schweißschritt durchgeführt wird, um den feststehenden Teil des Biegeinstruments für ein Endoskop an den äußeren Umfang des Spitzenendes der Hülle zu schweißen.

3. Verfahren nach Anspruch 1, wobei der feststehende Teil und der Schiebeteil ringförmig oder zylindrisch ausgebildet werden und bestimmte Abschnitte an gegenüberliegenden Endflächen des feststehenden Teils und des Schiebeteils durch den verlängerten Verbindungsteil verbunden sind.

4. Verfahren nach einem der vorhergehenden Ansprüche, wobei sich das Endoskop zur Einführung in einen Gastrostomiekatheter eignet, der mit einem röhrenförmigen, mit einem inneren Durchgangsloch ausgebildeten Teil und einem mageninternen feststehenden Teil versehen ist, der mit dem Spitzenende des röhrenförmigen Teils verbunden ist, wobei das Spitzenende des Durchgangslochs des röhrenförmigen Teils offen bleibt, und der im Bereich des Spitzenendes des Durchgangslochs mit einem Eingriffsteil versehen ist, wobei der röhrenförmige Teil in einer zwischen der Hautoberfläche eines Patienten und der Innenfläche der Magenwand ausgebildeten Magenfistel positioniert wird und der Gastrostomiekatheter in die Magenfistel gelegt wird, wobei der mageninterne feststehende Teil innerhalb des Magens positioniert ist, und wobei der feststehende Teil zusammen mit dem Endoskop durch das Innere des Gastrostomiekatheters gehen kann und der Schiebeteil durch das Durchgangsloch des Gastrostomiekatheters gehen kann, aber den Eingriffsteil des mageninternen feststehenden Teils so in Eingriff nimmt, dass er nicht durch den mageninternen feststehenden Teil gehen kann.

5. Verfahren nach einem der vorhergehenden Ansprüche, wobei das Endoskop mit einer Hülle bedeckt ist und das Biegeinstrument für ein Endoskop am Endoskop angebracht wird, wobei die Hülle dazwischen liegt.

6. Biegeinstrument (10) für ein Endoskop, das zum Anbringen an das Endoskop verwendet wird, um den Spitzenendenabschnitt des Endoskops zu biegen, wobei das Biegeinstrument für ein Endoskop einen feststehenden Teil (11), der am äußeren Umfang des Spitzenendes des Endoskops angebracht werden kann, einen Schiebeteil (12), der im Gebrauch weiter zum Basisende des Endoskops hin als der Abschnitt, an dem der feststehende Teil angebracht ist, schiebbar angebracht ist, und einen flexiblen Verbindungsteil (13) zum Verbinden bestimmter gegenüberliegender Abschnitte des feststehenden Teils und des Schiebeteils umfasst, wobei der feststehende Teil, der Schiebeteil und der Verbindungsteil einteilig ausgebildet sind und der Spalt zwischen dem feststehenden Teil und dem Schiebeteil durch den verlängerten Verbindungsteil beschränkt werden kann, so dass er die spezifische Länge nicht überschreitet, **dadurch gekennzeichnet, dass** der flexible Verbindungsteil durch Verlängern des Verbindungsteils auf eine bestimmte Länge mittels Ziehens gebildet ist und an seinem mittleren Abschnitt dünner als an jedem Endabschnitt ist und der Verbindungsteil, der feststehende Teil und der Schiebeteil einteilig geformt sind.

7. Biegeinstrument für ein Endoskop nach Anspruch 6, wobei der feststehende Teil und der Schiebeteil ringförmig oder zylindrisch sind und bestimmte Abschnitte an gegenüberliegenden Endflächen des feststehenden Teils und des Schiebeteils durch den flexiblen Verbindungsteil verbunden sind.

8. Biegeinstrument für ein Endoskop nach einem der Ansprüche 6 bis 7, in Kombination mit einem Endoskop (15) und einem Gastrostomiekatheter (25) zum Einführen des Endoskops in den Gastrostomiekatheter, wobei der Gastrostomiekatheter mit einem röhrenförmigen, mit einem inneren Durchgangsloch ausgebildeten Teil (27) und einem mageninternen feststehenden Teil (28) versehen ist, der mit dem Spitzenende des röhrenförmigen Teils verbunden ist, wobei das Spitzenende des Durchgangslochs des röhrenförmigen Teils offen bleibt, und der im Bereich des Spitzenendes des Durchgangslochs mit einem Eingriffsteil versehen ist, wobei der röhrenförmige Teil in einer zwischen der Hautoberfläche eines Patienten und der Innenfläche der Magenwand ausgebildeten Magenfistel positioniert ist und der Gastrostomiekatheter geeignet ist, in die Magenfistel gelegt zu werden, wobei der mageninterne feststehende Teil innerhalb des Magens positioniert ist, und
wobei der feststehende Teil zusammen mit dem Endoskop durch das Innere des Gastrostomiekatheters gehen kann und der Schiebeteil durch das Durchgangsloch des Gastrostomiekatheters gehen kann, aber den Eingriffsteil des mageninternen feststehenden Teils so in Eingriff nimmt, dass er nicht durch den mageninternen feststehenden Teil gehen kann.

9. Biegeinstrument für ein Endoskop nach einem der Ansprüche 6 bis 7, in Kombination mit einem Endoskop (15), wobei das Endoskop mit einer Hülle (14) bedeckt ist und das Biegeinstrument für ein Endoskop am Endoskop angebracht ist, wobei die Hülle dazwischen liegt.

## Revendications

1. Procédé de production d'un instrument de pliage (10) pour endoscope, qui est utilisé pour être attaché à un endoscope afin de plier la section d'extrémité de pointe de l'endoscope,
ledit procédé étant **caractérisé en ce qu'**il comporte :
une étape de moulage dans laquelle une partie fixe (11) qui est attachée à la périphérie extérieure de l'extrémité de pointe de l'endoscope, une partie coulissante (12) qui est attachée de manière coulissante plus près de l'extrémité de base de l'endoscope que la portion au niveau de laquelle la partie fixe est attachée, et une partie de liaison (13) servant à relier des portions spécifiques opposées de la partie fixe et de la partie coulissante, sont moulées d'une seule pièce par moulage par injection ; et
une étape d'étirage dans laquelle la partie de liaison du corps moulé, moulé au cours de l'étape de moulage, est formée en une partie de liaison flexible étendue servant à réduire l'espace entre la partie fixe et la partie coulissante de telle sorte qu'il ne dépasse pas la longueur spécifique, en étendant la partie de liaison jusqu'à une longueur spécifique par étirage de telle sorte que les deux portions d'extrémité de la partie de liaison étendue dans la direction longitudinale soient plus épaisses ou plus larges que la portion centrale de celle-ci.

2. Procédé selon la revendication 1, dans lequel l'endoscope est recouvert par une gaine (14) pendant l'utilisation, et le corps moulé, moulé au cours de l'étape de moulage, est moulé à partir du même matériau que le matériau constituant la gaine, et une étape de soudage est mise en oeuvre après l'étape d'étirage, afin de souder la partie fixe de l'instrument de pliage pour endoscope à la périphérie extérieure de l'extrémité de pointe de la gaine.

3. Procédé selon la revendication 1, dans lequel la partie fixe et la partie coulissante sont formées de manière à être annulaires ou cylindriques, et des portions spécifiques sur des faces d'extrémité opposées de la partie fixe et de la partie coulissante sont réunies par la partie de liaison étendue.

4. Procédé selon l'une quelconque des revendications précédentes, dans lequel l'endoscope est approprié pour être inséré dans un cathéter de gastrostomie, qui est pourvu d'une partie tubulaire formée avec un trou traversant interne, et d'une partie fixe à l'intérieur de l'estomac qui est reliée à l'extrémité de pointe de la partie tubulaire, l'extrémité de pointe du trou traversant de la partie tubulaire restant ouverte, et qui est pourvue d'une partie d'engagement dans la région de l'extrémité de pointe du trou traversant ; la partie tubulaire étant positionnée dans une fistule gastrique formée entre la surface de la peau d'un patient et la surface intérieure de la paroi de l'estomac, et le cathéter de gastrostomie étant fabriqué à demeure dans la fistule gastrique avec la partie fixe à l'intérieur de l'estomac positionnée à l'intérieur de l'estomac ; et la partie fixe pouvant passer à travers l'intérieur du cathéter de gastrostomie conjointement avec l'endoscope, et la partie coulissante pouvant passer à travers le trou traversant du cathéter de gastrostomie mais s'engageant avec la partie d'engagement de la partie fixe à l'intérieur de l'estomac de manière à ce qu'elle ne puisse pas passer à travers la partie fixe à l'intérieur de l'estomac.

5. Procédé selon l'une quelconque des revendications précédentes, dans lequel l'endoscope est recouvert par une gaine, et l'instrument de pliage pour endoscope est attaché à l'endoscope par interposition de la gaine.

6. Instrument de pliage (10) pour endoscope, qui est utilisé pour être attaché à un endoscope afin de plier la section d'extrémité de pointe de l'endoscope,
ledit instrument de pliage pour endoscope comprenant une partie fixe (11) qui peut être attachée à la périphérie extérieure de l'extrémité de pointe de l'endoscope, une partie coulissante (12) qui, pendant l'utilisation, est attachée de manière coulissante plus près de l'extrémité de base de l'endoscope que la portion au niveau de laquelle la partie fixe est attachée, et une partie de liaison flexible (13) servant à relier des portions spécifiques opposées de la partie fixe et de la partie coulissante, la partie fixe, la partie coulissante et la partie de liaison étant formées d'une seule pièce et l'espace entre la partie fixe et la partie coulissante pouvant être réduit par la partie de liaison étendue de telle sorte qu'il ne dépasse pas la longueur spécifique, **caractérisé en ce que** la partie de liaison flexible est formée en étendant la partie de liaison jusqu'à une longueur spécifique par étirage et **en ce qu'**elle est plus mince au niveau d'une portion centrale de celle-ci qu'à chaque portion d'extrémité, la partie de liaison, la partie fixe et la partie coulissante étant moulées d'une seule pièce.

7. Instrument de pliage pour endoscope selon la revendication 6, dans lequel la partie fixe et la partie coulissante sont annulaires ou cylindriques, et des portions spécifiques sur des faces d'extrémité opposées de la partie fixe et de la partie coulissante sont réunies par la partie de liaison flexible.

8. Instrument de pliage pour endoscope selon l'une quelconque des revendications 6 et 7, en combinaison avec un endoscope (15) et un cathéter de gastrostomie (25) pour l'insertion de l'endoscope dans le cathéter de gastrostomie, le cathéter de gastrostomie étant pourvu d'une partie tubulaire (27) formée avec un trou traversant interne, et d'une partie fixe à l'intérieur de l'estomac (28), qui est reliée à l'extrémité de pointe de la partie tubulaire, l'extrémité de pointe du trou traversant de la partie tubulaire restant ouverte, et qui est pourvue d'une partie d'engagement dans la région de l'extrémité de pointe du trou traversant ; la partie tubulaire étant positionnée dans une fistule gastrique formée entre la surface de la peau d'un patient et la surface intérieure de la paroi de l'estomac, et le cathéter de gastrostomie étant approprié pour être inséré à demeure dans la fistule gastrique, la partie fixe à l'intérieur de l'estomac étant positionnée à l'intérieur de l'estomac ; et
la partie fixe pouvant passer à travers l'intérieur du cathéter de gastrostomie conjointement avec l'endoscope, et la partie coulissante pouvant passer à travers le trou traversant du cathéter de gastrostomie mais s'engageant avec la partie d'engagement de la partie fixe à l'intérieur de l'estomac de telle sorte qu'elle ne puisse pas passer à travers la partie fixe à l'intérieur de l'estomac.

9. Instrument de pliage pour endoscope selon l'une quelconque des revendications 6 et 7 en combinaison avec un endoscope (15), dans lequel l'endoscope est recouvert par une gaine (14), et l'instrument de pliage pour endoscope est attaché à l'endoscope par interposition de la gaine.
